# EUROPEAN PATENT SPECIFICATION

(11) **EP 2 928 324 B1**
(45) Date of publication and mention of the grant of the patent: **20.07.2016**
(21) Application number: 14718502.9
(22) Date of filing: 17.03.2014
(51) Int. Cl.: A23L 29/00, A23L 33/17, A61K 38/01, A61K 36/258, A61K 36/42, A61K 36/254

(54) **COMPOSITION AND USE OF A DIETARY SUPPLEMENT**
ZUSAMMENSETZUNG UND VERWENDUNG EINER DIÄTETISCHEN NAHRUNGSERGÄNZUNG
COMPOSITION ET UTILISATION D'UN COMPLÉMENT ALIMENTAIRE

(30) Priority: 04.04.2013 BE 201300237
(43) Date of publication of application: 14.10.2015
(73) Proprietor: New Matrix, Besloten Vennootschap Met Beperkte Aansprakelijkheid, 3500 Hasselt (BE)
(72) Inventor: JACQUET, Baudry, Maria, G., B-3500 Hasselt (BE)
(74) Representative: Donné, Eddy
(86) International application number: PCT/BE2014/000012
(87) International publication number: WO 2014/169357

(56) References cited:
- DATABASE EMBASE [Online] ELSEVIER SCIENCE PUBLISHERS, AMSTERDAM, NL; March 2005 (2005-03), ADLER M: "Red ginseng - A Far Eastern "multitalent" in portrait of the daily practice", XP002717032, Database accession no. EMB-2005196480 & ARZTEZEITSCHRIFT FUR NATURHEILVERFAHREN UND REGULATIONSMEDIZIN 200503 DE, vol. 46, no. 3, March 2005 (2005-03), pages 138-145, ISSN: 1614-8339
- DATABASE WPI Week 200929 Thomson Scientific, London, GB; AN 2009-G08798 XP002717033, & KR 100 880 537 B1 (SYSPHARM CO LTD) 28 January 2009 (2009-01-28)
- MICLO L ET AL: "Characterization of alph-casozepine, a tryptic peptide from bovine alphas1-casein with benzodiazepine-like activity", FASEB JOURNAL, FED. OF AMERICAN SOC. FOR EXPERIMENTAL BIOLOGY, US, vol. 15, no. 10, 1 August 2001 (2001-08-01), pages 1780-1792, XP002481109, ISSN: 0892-6638 [retrieved on 2001-06-08]
- CAKIR-KIEFER CELINE ET AL: "In Vitro Digestibility of alpha-Casozepine, a Benzodiazepine-like Peptide from Bovine Casein, and Biological Activity of Its Main Proteolytic Fragment", JOURNAL OF AGRICULTURAL AND FOOD CHEMISTRY, vol. 59, no. 9, May 2011 (2011-05), pages 4464-4472, XP002717034, ISSN: 0021-8561

## Description

The present invention relates to a composition of a dietary supplement and the use and a dosage method thereof.

More specifically, the invention is intended to combat the symptoms of a professional exhaustion syndrome, also called "burnout" syndrome.

It is known that continually making extraordinary efforts on a professional level can lead to symptoms of physical and mental exhaustion, that can be summarised by the name "burnout" syndrome.

This burnout syndrome can be defined as a condition that is characterised by a set of signs, symptoms and behavioural changes in the professional environment. In general morphological, functional or biochemical changes are observed in people who suffer from it. As this condition is the result of a permanent and continuous exposure to professional stress, this burnout syndrome is placed in the category of psychosocial occupational hasards.
In order to combat these burnout symptoms, people sometimes resort to dietary supplements such as taking vitamins or minerals, but generally without obtaining a quick and adequate response.

DATABASE EMBASE [Online] ELSEVIER SCIENCE PUBLISHERS, AMSTERDAM, NL; March 2005 (2005-03), ADLER M: "Red ginseng - A Far Eastern "multitalent" in portrait of the daily practice", XP002717032, Database accession no. EMB-2005196480 ; & ARZTEZEITSCHRIFT FUR NATURHEILVERFAHREN UND REGULATIONSMEDIZIN 200503 DE,vol. 46, no. 3, March 2005 (2005-03), pages 138-145,ISSN: 1614-8339 disclosesthat Red ginseng (Panax ginseng) is indicated for various diseases, including burnout syndrome.

The purpose of the present invention is to provide a solution to the aforementioned and other disadvantages by providing a dietary supplement that enables the symptoms of burnout to be fought.

The following table shows the active ingredients of the dietary supplement, shown as a quantity in mg/coated tablet, whereby a full coated tablet or pill weighs 640 mg and the normal administered dose is 2 coated tablets per day.

**Table I. Active ingredients in the dietary supplement.**

| Scientific name | Trivial name | Quantity mg / pill | Function |
|---|---|---|---|
| α - casein tryptic hydrolysate | Casozepine | 75 mg | Active ingredient |
| Eleuthero-cocccus senticosus L. or Panax Ginseng | Siberian or Asian Ginseng Root Powder | 50 mg | Active ingredient |
| 2-aminoethanesulfonic acid | Taurine | 50 mg | Active ingredient |
| Cucumis melo L. | Extramel Lyophilised Melon Extract | 5 mg | Active ingredient |

The following table shows the other ingredients, in a 640 mg coated tablet, together with the function that they have in the dietary supplement.

**Table II: non-active ingredients in the dietary supplement.**

| Scientific name | Trivial name | Quantity mg / pill | Function |
|---|---|---|---|
| Microcrystalline cellulose | Cellulose | 400 mg | Thickening agent |
| Mg-stearate | Magnesium stearate | 20 mg | Anticlotting agent |
| SiO2 | Silicon dioxide | 20 mg | Anticlotting agent |
| Vegetable oil | Dehydrogenated vegetable oil | 10 mg | Glazing agent |
| Hypromellose | Hypromellose | 3 mg | Filler |
| Polydextrose | Polydextrose | 3 mg | Glazing agent |
| TiO2 | Titanium dioxide | 4.15 mg | Colouring agent |
| Propylene glycol | Propylene glycol E1520 | 0.35 mg | Glazing agent |
| Riboflavin-5-phosphate | Riboflavin-5-phosphate E101 | 0.05 mg | Colouring agent |
| Carmine | Carmine E120 | 0.03mg | Colouring agent |

The present invention concerns a dietary supplement, consisting of a mixture of at least α-casein tryptic hydrolysate, Siberian or Asian Ginseng root powder and lyophilised melon extract.

Preferably taurine is also added to the composition as a fourth active ingredient.

The present invention also concerns the use of a dietary supplement, with at least α-casein tryptic hydrolysate, Siberian or Asian Ginseng root powder and lyophilised melon extract for fighting burnout syndrome.

In order to evaluate the efficacy of this dietary supplement against burnout and to test the tolerance to it, a clinical trial was organised in the department of clinical pharmacology of the University of Bordeaux over a period of one year.

It was a randomised, double-blind test on two parallel groups, in which 87 volunteers participated. This means that
- half of the volunteers were given the dietary supplement according to the invention. We refer to this product hereinafter as the "verum".
- the other half of the volunteers received a placebo product, i.e. a product without pharmacological effect. We refer to this product hereinafter as the "placebo".
- the volunteer did not know whether he was taking the "placebo" or the "verum" product.
- the trial physician did not know whether he was giving a placebo or a verum product, which is why the test is called a double-blind test.

In this test the "verum" product was administered orally every morning in the form of two 640 mg coated tablets with the above composition, and this every morning for 84 days.

The products were distributed according to a predetermined list drawn up by the centre of statistical analysis, which enabled a homogeneous distribution of the "placebo" and "verum": this is the 'randomisation'.

This list, which indicates which volunteer numbers had to be given a "placebo" and which numbers a "verum", could only be made known after the definitive results of the test had been given to the analysis centre, so that the double-blind nature was safeguarded.

This methodology is used most frequently for the clinical tests of dietary supplements. For the tests concerning this range of products, this is almost a requirement to obtain the approval of the ministerial authorities, at least in France.

The methodology comprises the following steps:
- 87 volunteers presenting clinical signs of burnout were included in the test (55 women and 32 men);
- they all have a profession in which they come into direct contact with patients or pupils or customers. This requirement of direct contact with patients, pupils or customers is to do with the fact that one of the international validated scales used in this test has been specifically designed for this type of profession and is not suitable for professions that do not have direct contact with these groups of patients, pupils or customers, i.e. the MBI-HSS scale;
- each volunteer was seen and examined up to three times: D0, D42 and D84, i.e. on day 0, after 42 days and after 84 days of treatment. At each examination measurements were taken of:

- the BMS-10 burnout scale: the abridged version of Maslach and Pines. Ten items are scored from 1 to 7 according to their frequency. These scores are determined by adding up the value of each item and dividing by ten. They thus vary from 1 to 7. In order to be included in this test programme, the score must be at least 4, which confirms the existence of burnout.
- the Maslach burnout inventory scale (MBI-HSS): this comprises 22 items that are scored from 0 to 6 according to their frequency, and which are divided into three groups:
   * burnout (9 items): varies between 0 and 54,
   * depersonalisation (5 items): between 0 and 30,
   * the completion of tasks (8 items): between 0 and 48.
- the Beck depression scale: this comprises 21 items that are scored between 0 and 3 according to their intensity. The score thus varies between 0 and 63. It enables the importance of the depression to be quantified that results from the burnout and is associated with it.

The results of these three scales were statistically processed to analyse the following:
* the development of each item within each group and this for the administration of the verum and the placebo;
* the comparison of the values of the scores between the verum and placebo groups;
* the table mentioned below only gives a summary of the statistical analysis, in which only the average values and the conclusions of statistical significance are reported.

The differences between the scores on day 0 and day 84 were calculated statistically for the individual scores of each volunteer and not for the average scores. The differences between day 0 and 48 are thus not differences of averages but averages of the differences.

To this end the score on day 0 was subtracted from the scores after 84 days for each volunteer, after which the average of all these differences was calculated.

In order to present the still unpublished results of this double-blind study, they are explained in the accompanying diagram, as an example without any limiting nature:

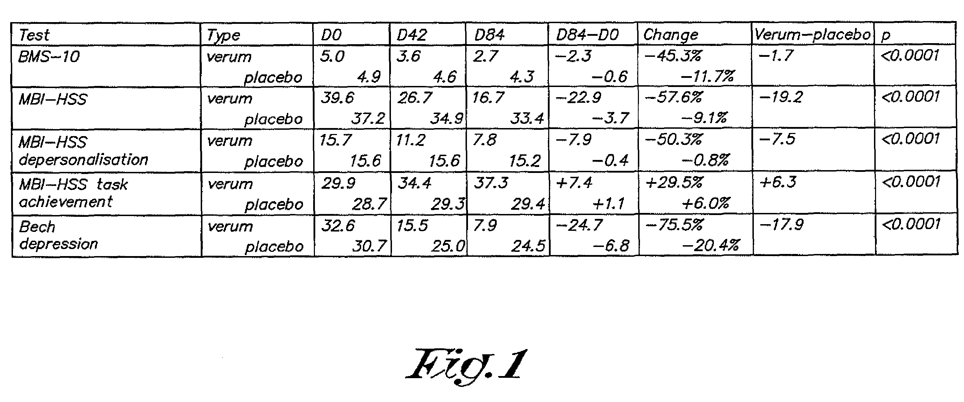

Figure 1 shows in tabular form the measured results of the effects of the composition according to the invention on burnout syndrome.

Figure 1 shows the measured data in tabular form and this for five test methods that are identified in the first column, which each yield a score relating to the burnout syndrome. The second column indicates whether the test concerned was taken after the administration of the dietary supplement according to the invention of the "verum" type, or after the administration of a "placebo" type without active ingredients. The third to fifth columns show the measured scores at the start of the test period (day 0), halfway through the test period (day 42) and at the end of the test period (day 84). The sixth column shows the difference of the measured value at the end of the test to the starting value of the test on day 0. The seventh column shows this difference as a percentage of the starting value.

The eighth column shows the difference for the "verum" score, from which the difference for the "placebo" score was subtracted, i.e. the net difference attributable to the dietary supplement. The ninth column shows the statistical significance of this net difference, which shows that the probability that the measured result is coincidental for all test methods is less than 1 in 10,000. This means that the measured effect of the dietary supplement on burnout syndrome can be called highly significant.

Also for other criteria than the said five test methods, the established effect of the composition according to the invention is statistically highly significant (p < 0.0001), such as for the following criteria:
- the quality of the professional life;
- the quality of family life;
- the quality of the night's sleep;
- the energy level of the patient.

Moreover, it was found that the tolerance to the dietary supplement turned out to be excellent.

The coated tablets can be administered daily over an extended period and the quantity of active ingredient per coated tablet can also vary between:
- 35 mg and 225 mg for the α - casein tryptic hydrolysate
- 25 mg and 150 mg for the Siberian or Asian Ginseng root powder;
- 2.5 mg and 15 mg for the lyophilised melon extract;
and optionally between:
25 mg and 150 mg for 2-aminosulfonic acid.

The present invention also concerns a dosing method for a dietary supplement with a composition according to the invention, whereby the dietary supplement is administered orally in the form of one or more coated tablets over a period of one or more weeks or months.

The present invention is by no means limited to the composition described as an example, but a dietary supplement according to the invention with at least the three active ingredients described in the first claim can be used in all kinds of compositions and doses, without departing from the scope of the invention.

## Claims

1. Composition of a dietary supplement, **characterised in that** it comprises a mixture of at least α-casein tryptic hydrolysate, Siberian or Asian Ginseng root powder, and lyophilised melon extract.

2. Composition according to claim 1, **characterised in that** 2-aminoethanesulfonic acid is added to the mixture.

3. Composition according to claim 1, **characterised in that** the quantity of active ingredient per coated tablet:
- is between 35 mg and 225 mg and preferably 75 mg for the α-casein tryptic hydrolysate;
- between 25 mg and 150 mg and preferably 50 mg for the Siberian or Asian Ginseng root powder;
- between 2.5 mg and 15 mg and preferably 5 mg for the lyophilised melon extract;

4. Composition according to claim 2, **characterised in that** the quantity of 2-aminoethanesulfonic acid per coated tablet:
- is between 25 mg and 150 mg and preferably 50 mg.

5. Dietary supplement, with at least α-casein tryptic hydrolysate, Siberian or Asian Ginseng root powder, and lyophilised melon extract for use in a method to combat burnout syndrome.

6. Dietary supplement for use according to claim 5, **characterised in that** the dietary supplement is administered orally in the form of one or more coated tablets per day over a period of one or more weeks or months.

## Patentansprüche

1. Zusammensetzung eines Nahrungsergänzungsmittels, **dadurch gekennzeichnet, dass** es ein Gemisch von mindestens tryptischem Hydrolysat von α-Casein, sibirischem oder asiatischem Ginsengwurzelpulver und gefriergetrocknetem Melonenextrakt umfasst.

2. Zusammensetzung nach Anspruch 1, **dadurch gekennzeichnet, dass** dem Gemisch 2-Aminoethansulfonsäure zugesetzt ist.

3. Zusammensetzung nach Anspruch 1, **dadurch gekennzeichnet, dass** die Menge an aktivem Bestandteil per Dragee:
- zwischen 35 mg und 225 mg und bevorzugt 75 mg für das tryptische Hydrolysat von α-Casein;
- zwischen 25 mg und 150 mg und bevorzugt 50 mg für das sibirische oder asiatische Ginsengwurzelpulver;
- zwischen 2,5 mg und 15 mg und bevorzugt 5 mg für den gefriergetrockneten Melonenextrakt beträgt;

4. Zusammensetzung nach Anspruch 2, **dadurch gekennzeichnet, dass** die Menge an 2-Aminoethansulfonsäure per Dragee:
- zwischen 25 mg und 150 mg und bevorzugt 50 mg beträgt.

5. Nahrungsergänzungsmittel, mit mindestens tryptischem Hydrolysat von α-Casein, sibirischem oder asiatischem Ginsengwurzelpulver und gefriergetrocknetem Melonenextrakt, zur Anwendung in einem Verfahren zur Bekämpfung von Bumout-Syndrom.

6. Nahrungsergänzungsmittel zur Anwendung nach Anspruch 5, **dadurch gekennzeichnet, dass** das Nahrungsergänzungsmittel oral in Form eines oder mehrerer Dragees pro Tag über einen Zeitraum von einer oder mehr Wochen oder Monaten verabreicht wird.

## Revendications

1. Composition d'un complément alimentaire, **caractérisée en ce qu'**elle comprend un mélange contenant au moins un hydrolysat trypsique de caséine alpha, de la poudre de racine de ginseng de Sibérie ou d'Asie et de l'extrait de melon lyophilisé.

2. Composition selon la revendication 1, **caractérisée en ce qu'**on ajoute au mélange de l'acide 2-aminoéthanesulfonique.

3. Composition selon la revendication 1, **caractérisée en ce que** la quantité d'ingrédient actif par comprimé enrobé :
- se situe entre 35 mg et 225 mg et de préférence s'élève à 75 mg pour l'hydrolysat trypsique de caséine alpha ;
- se situe entre 25 mg et 150 mg et de préférence s'élève à 50 mg pour la poudre de racine de ginseng de Sibérie ou d'Asie ;
- se situe entre 2,5 mg et 15 mg et de préférence s'élève à 5 mg pour l'extrait de melon lyophilisé.

4. Composition selon la revendication 2, **caractérisée en ce que** la quantité de l'acide 2-aminoéthanesulfonique par comprimé enrobé :
- se situe entre 25 mg et 150 mg et s'élève de préférence à 50 mg.

5. Complément alimentaire comprenant au moins un hydrolysat trypsique de caséine alpha, de la poudre de racine de ginseng de Sibérie ou d'Asie et de l'extrait de melon lyophilisé pour son utilisation dans un procédé destiné à combattre le syndrome du burnout.

6. Complément alimentaire pour son utilisation selon la revendication 5, **caractérisé en ce que** le complément alimentaire est administré par voie orale sous la forme d'un ou de plusieurs comprimés enrobés par jour pendant un laps de temps d'une ou de plusieurs semaines ou d'un ou de plusieurs mois.
